# EUROPÄISCHE PATENTANMELDUNG

(11) **EP 1 290 993 A1**
(43) Veröffentlichungstag der Anmeldung: **12.03.2003**
(21) Anmeldenummer: 02020102.6
(22) Anmeldetag: 07.09.2002
(51) Int. Cl.: A61F 2/44

(54) **Implantat**

(30) Priorität: 10.09.2001 DE 10145668
(71) Anmelder: Aesculap AG & Co. KG, 78532 Tuttlingen (DE)
(72) Erfinder: Pfeiffer, Ulrike, 78570 Mühlheim (DE); Eckhof, Stephan, 78532 Tuttlingen (DE); Samyn, Thomas, 78315 Radolfzell-Güttingen (DE); Schneid, Susanne, 78532 Tuttlingen (DE); Schultz, Robert, 78532 Tuttlingen (DE); Schumacher, Jörg, 78532 Tuttlingen (DE); Struss, Jürgen, 78549 Spaichingen (DE)
(74) Vertreter: Böhme, Ulrich, Dr. Dipl.-Phys.

(57) **Zusammenfassung**

Um ein Implantat (116) zum Ersetzen mindestens eines mindestens teilweise zerstörten Wirbelkörpers und zum Einsetzen zwischen einem ersten und einem zweiten Wirbelkörper (112,114) einer Wirbelsäule, mit mindestens einem Bewegungselement (124,125) und mindestens einem Stützelement (120), wobei das mindestens eine Bewegungselement dem Stützelement und dem ersten und/oder dem zweiten Wirbelkörper zugeordnet ist zum Ermöglichen einer Bewegung des mindestens einen Stützelements relativ zu dem ersten und/oder dem zweiten Wirbelkörper, so zu verbessern, daß annähernd physiologische Bewegungsmöglichkeiten betroffener Bewegungssegmente wiederhergestellt werden können und es besonders leicht eingesetzt werden kann und seine Position nach dem Einsetzen beibehält, wird vorgeschlagen, daß mindestens eine mit mindestens einem Wirbelbogen (118) der Wirbelsäule verbundene Fixiervorrichtung (126) vorgesehen ist zum Fixieren des mindestens einen Stützelements an dem mindestens einen Wirbelbogen.

## Beschreibung

Die Erfindung betrifft ein Implantat zum Ersetzen mindestens eines mindestens teilweise zerstörten Wirbelkörpers eines Wirbels und zum Einsetzen zwischen einem ersten und einem zweiten Wirbelkörper einer Wirbelsäule, mit mindestens einem Bewegungselement und mindestens einem Stützelement, wobei das mindestens eine Bewegungselement dem mindestens einen Stützelement und dem ersten und/oder dem zweiten Wirbelkörper zugeordnet ist zum Ermöglichen einer Bewegung des Stützelements relativ zu dem ersten und/oder dem zweiten Wirbelkörper.

Implantate der eingangs beschriebenen Art werden eingesetzt, um einen zerstörten oder auch nur teilweise zerstörten Wirbelkörper eines Wirbels der Wirbelsäule zu ersetzen. Das Ziel dabei ist es, annähernd physiologische Bewegungsmöglichkeiten betroffener Bewegungssegmente einschließlich damit verbundener dorsaler Strukturen beizubehalten beziehungsweise wiederherzustellen. Es soll mit einem solchen Implantat insbesondere vermieden werden, benachbarte Wirbelkörper mittels des Implantats zu fusionieren, also diese gerade nicht zu versteifen.

Als vorteilhaft hat es sich bei einer solchen, bewegungserhaltenden Maßnahme erwiesen, daß Bandscheiben benachbarter Segmente geschont werden. Sie müssen eine fehlende Beweglichkeit eines versteiften Segments nicht kompensieren, was bei üblichen Fusionseingriffen der Fall wäre. Dadurch kann eine frühzeitige Degeneration dieser Segmente infolge von Überbeanspruchung vermieden werden.

Als nachteilig hat sich bei Implantaten der eingangs beschriebenen Art erwiesen, daß sie nur mit einem äußerst großen operativen Aufwand eingesetzt werden können. Darüber hinaus ergeben sich Schwierigkeiten, weil das Implantat unter Belastung die gewünschte optimale Position zwischen dem ersten und zweiten Wirbelkörper nicht immer beibehält.

Daher ist es Aufgabe der vorliegenden Erfindung, ein Implantat der gattungsgemäßen Art so zu verbessern, daß annähernd physiologische Bewegungsmöglichkeiten betroffener Bewegungssegmente wiederhergestellt werden können und es besonders leicht eingesetzt werden kann und seine Position nach dem Einsetzen beibehält.

Diese Aufgabe wird bei einem Implantat der eingangs beschriebenen Art erfindungsgemäß dadurch gelöst, daß mindestens eine mit mindestens einem Wirbelbogen der Wirbelsäule verbundene Fixiervorrichtung vorgesehen ist zum Fixieren des Stützelements an dem mindestens einen Wirbelbogen.

Das Implantat wird nach dem Ausräumen der zerstörten Strukturen mit Hilfe geeigneter Instrumente in den ausgeräumten Bereich eingebracht. Es besteht insgesamt aus im wesentlichen mindestens drei Teilen, dem mindestens einen Stützelement, dem mindestens einen Bewegungselement und der mindestens einen Fixiervorrichtung. Das Stützelement besitzt nach der Implantation eine steife, im wesentlichen nicht verformbare Struktur, deren Flexibilität vernachlässigbar klein sein soll. Es ersetzt damit im wesentlichen den Wirbelkörper des Wirbels. Demgegenüber weist das Bewegungselement eine Beweglichkeit auf, die eine Bewegung zwischen dem Stützelement und dem oder den benachbarten Wirbelkörpern ermöglicht. Mit der erfindungsgemäß vorgeschlagenen zusätzlichen Fixiervorrichtung wird das Stützelement an dem mindestens einen verbliebenen Wirbelbogen des Patienten fixiert, so daß die gewünschte Position des Stützelements langfristig erhalten bleibt. Ein Vorteil dabei ist, daß durch diese besondere Fixiervorrichtung keine Einschränkung der Beweglichkeit zwischen dem Stützelement und den benachbarten Wirbelkörpern auftritt. Vielmehr sind die Bewegungselemente vollständig frei und können nahezu jede gewünschte Bewegung der Wirbelsäule ausführen und ausgleichen. Entscheidend ist einzig und allein, daß die Fixiervorrichtung eine steife, im wesentlichen nicht verformbare Verbindung zwischen dem Stützelement und dem mindestens einen verbleibenden Wirbelbogen gewährleistet. Insgesamt werden dadurch physiologische Gegebenheiten wiederhergestellt, insbesondere die Funktion von an der Wirbelsäule angeordneten Facettengelenken erhalten, und das Stützelement zusätzlich stabilisiert.

Besonders vorteilhaft ist, wenn die mindestens eine Fixiervorrichtung mit dem mindestens einen Stützelement und dem mindestens einen Wirbelbogen des teilweise zerstörten Wirbels verbindbar ist. Aufgrund der direkten Verbindung zwischen dem Stützelement und dem Wirbelbogen des teilweise zerstörten Wirbels wird ein in seiner Funktionsweise für den ursprünglichen intakten Wirbel nahezu gleichwertiger Ersatz geschaffen. Wie beim ursprünglichen Wirbel wird ein im wesentlichen in sich biegesteifes Element, der Wirbelkörper, nachgebildet, das den ursprünglichen Wirbelkörper mit dem an diesem angeordneten Wirbelbogen nachbildet.

Vorzugsweise ist das mindestens eine Stützelement im wesentlichen biegesteif. Dadurch wird die Stabilität des Implantats insgesamt erhöht. Außerdem wird dem Stützelement eine von dem Bewegungselement abweichende Funktion zugeordnet.

Günstig ist es, wenn das mindestens eine Stützelement einen Hohlraum aufweist. Dadurch wird einerseits der Materialbedarf verringert, andererseits wird es ermöglicht, den Hohlraum des Stützelements mit einer für den jeweiligen Fall am besten geeigneten Substanz zu füllen, beispielsweise Knochenspänen und/oder Knochenzement.

Gemäß einer bevorzugten Ausführungsform der Erfindung kann vorgesehen sein, daß das mindestens eine Stützelement eine mit einer aushärtbaren Substanz füllbare Hülle umfaßt. Durch die aushärtbare Substanz wird die Biegesteifigkeit des Stützelements zusätzlich erhöht.

Vorteilhaft ist es, wenn die Hülle biegeschlaff und/oder flexibel ist. Mit einer solchen Hülle läßt sich ein der physiologischen Form eines Wirbelkörpers optimal angepaßtes Implantat modellieren. Insbesondere lassen sich Neigungen von an benachbarten Wirbelkörpern angrenzenden Kontaktflächen des Stützelements beliebig und einfach einstellen. Erst wenn die aushärtbare Substanz in die Hülle eingefüllt ist, wird das Stützelement endgültig geformt.

Vorzugsweise ist die aushärtbare Substanz Knochenzement. Dieser läßt sich besonders leicht modellieren und weist die erforderlichen Festigkeitseigenschaften auf.

Gemäß einer weiteren bevorzugten Ausführungsform der Erfindung kann vorgesehen sein, daß die mindestens eine Fixiervorrichtung mindestens ein Fixierelement umfaßt. Dieses kann grundsätzlich beliebig gestaltet sein, jedoch ist es mindestens mit dem Stützelement und/oder dem verbleibenden Wirbelbogen verbindbar.

Besonders vorteilhaft ist es, wenn das mindestens eine Fixierelement mindestens eine Schraube, eine Pedikelschraube, einen Stift, einen Haken oder einen Dübel umfaßt. Derartige Fixierelemente lassen sich besonders einfach am mindestens einen Wirbelbogen anbringen und/oder mit dem Stützelement verbinden.

Vorzugsweise ist der mindestens eine Dübel in einen Hohlraum des mindestens einen Stützelements einsetzbar und die mindestens eine Schraube mit dem mindestens einen Dübel verbindbar. Durch den sich in dem Hohlraum während des Eindrehens der Schraube spreizenden Dübel wird eine besonders gute Verbindung zwischen der Schraube und dem Stützelement ermöglicht.

Einen besonders guten Halt erhält das Stützelement, wenn das mindestens eine Fixierelement einen Blindniet umfaßt.

Um eine besonders sichere und stabile Verbindung zu erhalten, ist es vorteilhaft, wenn das mindestens eine Fixierelement spreizbar ist und wenn es insbesondere einen spreizbaren Stift umfaßt. Es können aber auch spreizbare Schrauben, Haken und insbesondere auch Dübel vorgesehen sein, die beispielsweise in eine Ausnehmung des Stützelements eindringen und dasselbe durch Aufspreizen zumindest teilweise hinterschneiden können.

Bei einer weiteren bevorzugten Ausführungsform der Erfindung kann ferner vorgesehen sein, daß die mindestens eine Fixiervorrichtung mindestens einen Drahtstift mit mindestens zwei relativ zum mindestens einen Drahtstift in einer Fixierstellung in Längsrichtung des mindestens einen Drahtstifts festlegbaren Anschlagelemente umfaßt. Der Drahtstift kann beispielsweise das Stützelement durchdringen oder auf andere Weise mit diesem in Verbindung stehen. Die beiden Anschlagelemente dienen dazu, den Drahtstift einerseits am Stützelement, andererseits am verbleibenden Wirbelbogen abzustützen.

Vorzugsweise ist mindestens eines der mindestens zwei Anschlagelemente in einer Anlegestellung relativ zum mindestens einen Drahtstift bewegbar. Damit läßt sich auf einfache Weise das Stützelement relativ zum mindestens einen verbleibenden Wirbelbogen verspannen, indem das eine Anschlagelement vorzugsweise in Längsrichtung des Drahtstifts bewegt wird.

Um eine besonders gute und sichere Verbindung zu erzielen, kann es vorteilhaft sein, wenn der mindestens eine Drahtstift mit einem Gewinde versehen ist. Damit läßt sich der Abstand der beiden Anschlagelemente stufenlos einstellen.

Bei einer weiteren vorteilhaften Ausführungsform der Erfindung kann vorgesehen sein, daß der mindestens eine Drahtstift mit Rastelementen versehen ist. Diese sind besonders einfach herzustellen und ermöglichen eine Bewegung eines Anschlagelements relativ zum Drahtstift, ohne daß das Anschlagelement gedreht werden muß.

Zur Vermeidung von Verletzungen ist es besonders günstig, wenn die mindestens zwei Anschlagelemente tellerförmig sind.

Bei einer weiteren vorteilhaften Ausführungsform der Erfindung kann vorgesehen sein, daß das mindestens eine Fixierelement in Richtung auf den Wirbelbogen hakenförmig gekrümmt ist und mindestens einen am Wirbelbogen angeordneten Pedikel und/oder Dornfortsatz und/oder Querfortsatz im eingesetzten Zustand mindestens teilweise umschließt. Mit dieser Ausgestaltung wird das Stützelement fixiert, indem das Fixierelement praktisch am mindestens einen verbleibenden Wirbelbogen eingehakt wird. Seine Form ist so vorgesehen, daß sich das Fixierelement optimal an den verbleibenden Wirbelbogen anpaßt.

Eine besonders stabile Verbindung zwischen dem Stützelement und dem mindestens einen verbleibenden Wirbelbogen ergibt sich, wenn das mindestens eine hakenförmig gekrümmte Fixierelement den mindestens einen Pedikel und/oder Dornfortsatz und/oder Querfortsatz vollständig umgibt.

Eine weitere vorteilhafte Ausführungsform der Erfindung sieht vor, daß die mindestens eine Fixiervorrichtung ein mit einer Befestigungselementaufnahme versehenes Band umfaßt. Das Band kann beispielsweise das Stützelement und/oder den mindestens einen verbleibenden Wirbelbogen umgeben. Mittels der vorgesehenen Befestigungselementaufnahme läßt sich das Band am Stützelement und/oder am verbleibenden Wirbelbogen mit einem zusätzlichen Befestigungselement, das beispielsweise auch ein oben genanntes Fixierelement sein kann, festlegen.

Vorzugsweise kann vorgesehen sein, daß die mindestens eine Fixiervorrichtung eine das mindestens eine Stützelement umschließende Schelle umfaßt. Eine derartige Schelle kann besonders einfach am Stützelement festgelegt werden. Insbesondere kann sie auch eine Fixierelementaufnahme aufweisen.

Besonders vorteilhaft ist es, wenn die Schelle in einer Anlegestellung relativ zum mindestens einen Stützelement verschiebbar und in einer Fixierstellung zum mindestens einen Stützelement festlegbar ist. Dadurch läßt sich die Schelle in der Anlegestellung in eine gewünschte Position bringen und in der Fixierstellung relativ zum Stützelement in dieser Position festlegen. Die Schelle bildet somit ein Verbindungsglied zwischen dem Stützelement und dem mindestens einen verbleibenden Wirbelbogen, an welchem sie beispielsweise direkt oder mittels eines Fixier- oder Befestigungselements festgelegt werden kann.

Um eine optimale Positionierung der Fixiervorrichtung relativ zum Stützelement und zum mindestens einen verbleibenden Wirbelbogen zu ermöglichen, ist es von Vorteil, wenn die mindestens eine Fixiervorrichtung mindestens ein bewegliches Lagerelement zum beliebigen Verschwenken des Fixierelements relativ zum mindesten einen Stützelement umfaßt.

Besonders vorteilhaft ist es, wenn das mindestens eine Lagerelement kugelförmig ist. Damit ist eine Rotation des Lagerelements relativ zum Stützelement um eine beliebige Raumachse möglich und es läßt sich damit beliebig relativ zum Stützelement orientieren.

Um eine optimale Positionierung des Implantats zu gewährleisten, ist es günstig, wenn das mindestens eine Lagerelement einen Absorptionskoeffizient für Röntgenstrahlen aufweist, der von dem des mindestens einen Stützelements abweicht. Damit wird während des Einsetzens des Implantats die Positionierung des Lagerelements beziehungsweise des Stützelements eindeutig erkennbar. Das Lagerelement kann daher gleichsam als eine Art Zielvorrichtung dienen, beispielsweise zum Einsetzen zusätzlicher Fixierelemente.

Bei einer weiteren vorteilhaften Ausführungsform der Erfindung kann vorgesehen sein, daß das mindestens eine Lagerelement eine mit einer Zentralbohrung versehene Kugel umfaßt. Dies ermöglicht es, beispielsweise ein Fixierelement durch die Zentralbohrung durchzuführen und mit der Kugel zu verbinden. So ist eine relative Bewegung des an der Kugel festgelegten beziehungsweise durchgeführten Fixierelements relativ zum Stützelement möglich.

Vorzugsweise kann vorgesehen sein, daß das mindestens eine Fixierelement ein Nahtmaterial umfaßt, das mit dem mindestens einen Wirbelbogen vernäh- oder verknotbar ist. Mit einem solchen Fixierelement läßt sich auf besonders einfache Weise das Stützelement am verbleibenden Wirbelbogen fixieren, und zwar, indem es mit diesem vernäht oder verknotet wird.

Um die Verbindung zwischen dem Stützelement und dem mindestens einen verbleibenden Wirbelbogen bei Verwendung von Nahtmaterial zu verbessern, ist es vorteilhaft, wenn die mindestens eine Fixiervorrichtung mindestens einen Knochenanker zum Fixieren des Nahtmaterials am Wirbelbogen umfaßt. Das Nahtmaterial wird auf diese Weise nicht mehr nur vernäht oder verknotet, sondern zusätzlich im Knochen des Wirbelbogens verankert.

Um eine Positionierung des Stützelements relativ zum mindestens einen verbleibenden Wirbelbogen zu verbessern, kann an dem mindestens einen Fixierelement ein auf einem Gewinde bewegbarer Anschlag vorgesehen sein. Mittels des Gewindes läßt sich eine Relativposition stufenlos einstellen. Eine solche Ausgestaltung ist insbesondere dann von Vorteil, wenn das Fixierelement in einen Hohlraum oder eine Ausnehmung des Stützelements eindringt.

Bei einer weiteren vorteilhaften Ausführungsform der Erfindung kann vorgesehen sein, daß das mindestens eine Fixierelement einen Fluidkanal zum Einführen eines Fluids in einen Hohlraum des mindestens einen Stützelements und/oder zum Einführen eines aushärtbaren Fluids zum Verankern des Fixierelements im Wirbelbogen umfaßt. Das Fixierelement hat dadurch eine Doppelfunktion: einerseits dient es zur Fixierung des Stützelements, andererseits zur Beschickung des Stützelements mit einem Fluid, beispielsweise flüssigem Knochenzement, der in den Hohlraum eingefüllt wird, um dort auszuhärten. Oder das Fixierelement wird selbst oder ergänzend über den Fluidkanal in den Wirbelbogen einzementiert.

Vorzugsweise umfaßt die mindestens eine Fixiervorrichtung mindestens eine am mindestens einen Stützelement vorgesehene Fixierelementaufnahme zum Aufnehmen des mindestens einen, am mindestens einen Wirbelbogen befestigbaren Fixierelements. Beispielsweise kann eine solche Fixierelementaufnahme durch eine an einer Schelle oder einem Band vorgesehene Gewindebohrung realisiert werden. Entscheidend ist, daß das Fixierelement mit dem Stützelement beziehungsweise einem weiteren Element der Fixiervorrichtung lösbar verbindbar ist.

Beispielsweise kann es vorteilhaft sein, wenn die mindestens eine Fixierelementaufnahme eine Gewindebohrung umfaßt.

Von Vorteil ist es ferner, wenn das mindestens eine Stützelement eine vom mindestens einen Wirbelbogen weg weisende Ausnehmung umfaßt. Dies ermöglicht es, daß ein Fixierelement in das Stützelement eindringen und an diesem festgelegt werden kann, beispielsweise über eine Mutter oder ein weiteres Verriegelungselement, das teilweise oder vollständig in die Ausnehmung eintauchen kann und somit nicht vom Stützelement absteht. Ferner kann diese Ausnehmung mit Knochenzement und/oder Knochenspänen ausmodelliert werden.

Zur Verbesserung der relativen Positionierung zwischen dem Stützelement und dem mindestens einen verbleibenden Wirbelbogen ist es günstig, wenn die Ausnehmung einen Anschlag für das mindestens eine Fixierelement umfaßt.

Um eine optimale physiologische Anpassung des Implantats an dem mindestens einen verbleibenden Wirbelbogen zu erreichen, ist es vorteilhaft, wenn das mindestens eine Bewegungselement eine mit einer dauerhaft fließfähigen Substanz gefüllte Hülle umfaßt. Damit wird eine bandscheibenähliche Funktion durch das Bewegungselement erzielt.

Gemäß einer weiteren vorteilhaften Ausführungsform der Erfindung ist das mindestens eine Bewegungselement aus einer teilweise elastischen Substanz hergestellt. Auch mit einer derartigen Ausgestaltung läßt sich eine künstliche Bandscheibe mit nahezu allen gewünschten physiologischen Eigenschaften verwirklichen.

Vorzugsweise umfaßt das mindestens eine Bewegungselement konkav oder konvex gekrümmte kalottenförmige Bereiche zur Bildung von Gleitflächen für daran anliegende Bereiche des Stützkörpers. Dadurch wird die Stabilität des künstlichen Wirbels zusätzlich erhöht, insbesondere eine zusätzliche Führung des Bewegungselements am Stützkörper geschaffen.

Um das Implantat auf einfache und sichere Weise zwischen benachbarte Wirbelkörper einzupassen, ist es günstig, wenn eine dem Implantat zugeordnete Zielvorrichtung vorgesehen ist. Damit läßt sich beim Einsetzen des Implantats die Position desselben ständig beobachten. Insbesondere ist es möglich, Fixierelemente oder weitere Teile des Implantats in einer gewünschten Weise relativ zu anderen Teilen des Implantats oder dem mindestens einen Wirbelbogen einzusetzen.

Besonders günstig ist es, wenn die Zielvorrichtung am Implantat angeordnet ist. Damit läßt sich die Position des Implantats besonders einfach bestimmen.

Grundsätzlich gibt es verschiedene Möglichkeiten, die Zielvorrichtung auszugestalten, vorzugsweise ist die Zielvorrichtung aus einem Material hergestellt, das einen Absorptionskoeffizienten für Röntgenstrahlen aufweist, der von dem des mindestens einen Stützelements abweicht. Damit läßt sich bei Bestrahlung mit Röntgenstrahlen die Zielvorrichtung besonders gut erkennen.

Um einen Kontrast bei der Abbildung des Implantats mittels Röntgenstrahlen zu erhöhen, ist es vorteilhaft, wenn das Implantat im wesentlichen aus für Röntgenstrahlen durchlässigem oder teilweise durchlässigem Material hergestellt ist. Damit kann aus der relativ zum Implantat bekannten Position der Zielvorrichtung auch die Position des Implantats bestimmt werden, wenn der Kontrast zwischen der abgebildeten Zielvorrichtung und dem abgebildeten Implantat ausreichend groß ist.

Die nachfolgende Beschreibung bevorzugter Ausführungsformen der Erfindung dient im Zusammenhang mit der Zeichnung der näheren Erläuterung. Es zeigen:
- Figur 1:: Eine Längsschnittansicht eines ersten Ausführungsbeispiels eines erfindungsgemäßen Implantats, das einen teilweise zerstörten Wirbelkörper eines Wirbels in einer Wirbelsäule eines Menschen ersetzt;
- Figur 2:: eine Längsschnittansicht ähnlich Figur 1 durch ein zweites Ausführungsbeispiel eines Implantats;
- Figur 3:: eine Längsschnittansicht ähnlich Figur 1 durch ein drittes Ausführungsbeispiel eines Implantats;
- Figur 4:: eine Längsschnittansicht ähnlich Figur 1 durch ein viertes Ausführungsbeispiel eines Implantats;
- Figur 5:: eine Längsschnittansicht ähnlich Figur 1 durch ein fünftes Ausführungsbeispiel eines Implantats;
- Figur 6:: eine Querschnittansicht durch ein sechstes Ausführungsbeispiel eines in einer Wirbelsäule eingesetzten Implantats;
- Figur 7:: eine Querschnittansicht ähnlich Figur 6 durch ein siebtes Ausführungsbeispiel eines Implantats;
- Figur 8:: eine Querschnittansicht ähnlich Figur 6 durch ein achtes Ausführungsbeispiel eines Implantats;
- Figur 9:: eine Querschnittansicht ähnlich Figur 6 durch ein neuntes Ausführungsbeispiel eines Implantats;
- Figur 10:: eine Längsschnittansicht ähnlich Figur 1 durch ein zehntes Ausführungsbeispiel eines Implantats;
- Figur 11:: eine Längsschnittansicht ähnlich Figur 1 durch ein elftes Ausführungsbeispiel eines Implantats;
- Figur 12:: eine Längsschnittansicht ähnlich Figur 1 durch ein zwölftes Ausführungsbeispiel eines Implantats;
- Figur 13:: eine Längsschnittansicht ähnlich Figur 1 durch ein dreizehntes Ausführungsbeispiel eines Implantats;
- Figur 14:: eine Längsschnittansicht ähnlich Figur 1 durch ein vierzehntes Ausführungsbeispiel eines Implantats;
- Figur 15:: eine Längsschnittansicht ähnlich Figur 1 durch ein fünfzehntes Ausführungsbeispiel eines Implantats;
- Figur 16:: eine Querschnittansicht ähnlich Figur 6 durch ein sechzehntes Ausführungsbeispiel eines Implantats;
- Figur 17:: eine Querschnittansicht ähnlich Figur 6 durch ein siebzehntes Ausführungsbeispiel eines Implantats;
- Figur 18:: eine Querschnittansicht ähnlich Figur 6 durch ein achtzehntes Ausführungsbeispiel eines Implantats;
- Figur 19:: eine Querschnittansicht ähnlich Figur 6 durch ein neunzehntes Ausführungsbeispiel eines Implantats;
- Figur 20:: eine Querschnittansicht ähnlich Figur 6 durch ein zwanzigstes Ausführungsbeispiel eines Implantats;
- Figur 21:: eine Seitenansicht eines Implantats aus Figur 2 mit einer ersten Variante einer daran angeordneten Zielvorrichtung; und
- Figur 22:: eine Ansicht ähnlich Figur 21 mit einer zweiten Variante einer am Implantat angeordneten Zielvorrichtung.

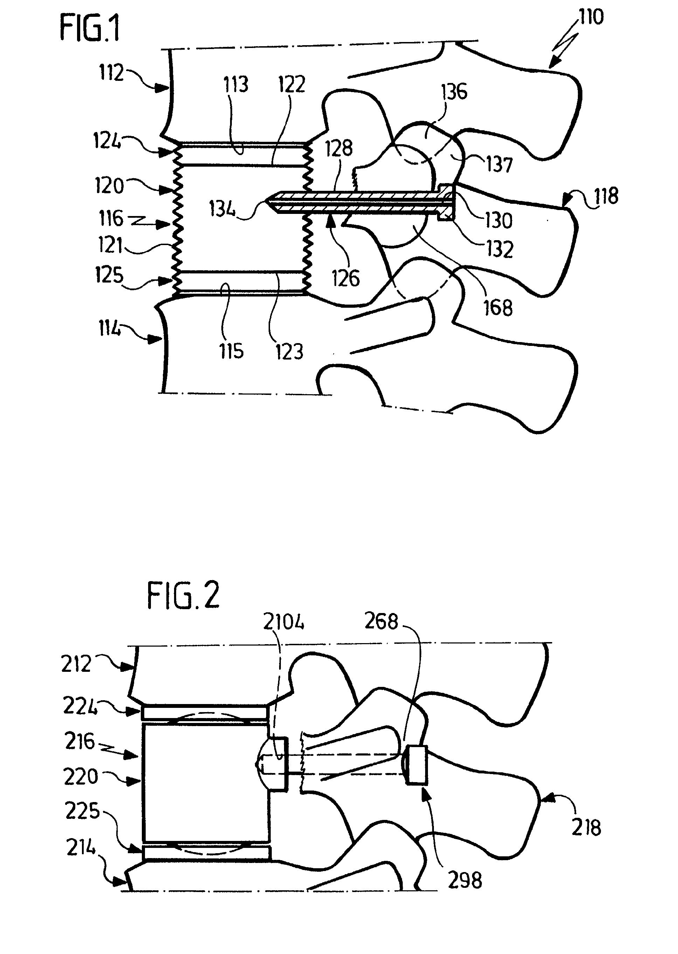

In Figur 1 ist schematisch ein Ausschnitt einer insgesamt mit dem Bezugszeichen 110 versehenen Wirbelsäule dargestellt, wobei zwischen zwei intakten Wirbelkörpern 112 und 114 ein Implantat 116 eingesetzt ist, das einen teilweisen zerstörten Wirbelkörper zumindest teilweise ersetzt.

Das Implantat 116 umfaßt im wesentlichen einen rotationssymmetrischen Stützkörper 120, der zwei in Richtung auf die Wirbelkörper 112 beziehungsweise 114 hin weisende Stirnflächen 122 und 123 aufweist. Zwischen diese und die benachbarten Wirbelkörper 112 und 114 ist jeweils ein als Bewegungselement wirkender beziehungsweise dienender, künstlicher Bandscheibenkörper 124 beziehungsweise 125 eingesetzt, welcher jeweils mit dem Stützkörper 120 verbunden ist.

Zur Fixierung des Stützkörpers 120 am Wirbelbogen 118 des teilweise zerstörten Wirbels ist eine Knochenschraube 126 vorgesehen, die längs eines Schafts 128 einen Fluidkanal 130 in Form einer Längsbohrung aufweist, der sich von einem Schraubenkopf 132 bis zur Schraubenspitze 134 erstreckt.

Das Implantat 116 wird eingesetzt, nachdem die teilweise zerstörten Strukturen des Wirbels entfernt worden sind, zu denen auch die an den benachbarten Wirbelkörpern 112 und 114 anliegenden Bandscheiben gehören. Der Stützkörper 120 mit den daran angeordneten Bandscheibenkörpern 124 und 125 wird an benachbarte Wirbelkörperflächen 113 und 115 angelegt und/oder befestigt.

Von dorsal nach ventral wird die Knochenschraube 126 durch den Wirbelbogen mit seinen verbliebenen Strukturen hindurchgeschraubt, insbesondere beispielsweise durch einen Pedikel 168, und zwar so weit, bis die Schraubenspitze 134 ins Innere des Stützkörpers 120 ragt. Der eine flexible Hülle 121 aufweisende Stützkörper 120 wird über den Fluidkanal 130 mit Knochenzement gefüllt. Bis zum Aushärten des Knochenzements muß das Implantat 116 in der gewünschten Form gehalten werden.

Alternativ ist es möglich, die Knochenschraube 126 erst nach dem Befüllen des Stützkörpers 120 in denselben einzuschrauben. Dies kann vor oder nach dem Aushärten des Knochenzements geschehen.

Die Bandscheibenkörper 124 und 125 sind entweder mit einer dauerhart fließfähigen oder einer elastischen Substanz gefüllt, beispielsweise einem Hydrogel.

Ist das Implantat 116 auf die oben beschriebene Weise fixiert, so bildet es in Verbindung mit dem Wirbelbogen 118 einen praktisch voll funktionsfähigen Wirbelersatz. Insbesondere ist es möglich, die Wirbelkörper 112 und 114 relativ zueinander zu bewegen, was bei einer Fusion über ein entsprechendes Implantat nicht der Fall wäre. Auch die beispielsweise von vom Wirbelbogen 118 beziehungsweise benachbarten Wirbelbögen abstehenden Facetten 136 und 137 gebildeten Facettengelenke im dorsalen Bereich der Wirbelbögen bleiben bei einem derartigen Implantat voll funktionsfähig.

In Figur 2 ist ein zweites Ausführungsbeispiel eines insgesamt mit dem Bezugszeichen 216 versehenen Implantats dargestellt, welches in analoger Weise wie im Zusammenhang mit dem Implantat 116 beschrieben, zwischen zwei benachbarte Wirbelkörper 212 und 214 eingesetzt wird.

Ein Stützkörper 220 wird an einem verbliebenen Rest eines Wirbels mittels zweier Pedikelschrauben 298 fixiert, wobei letztere jeweils einen Pedikel 268 durchdringen und in Gewindebohrungen 2104 eingeschraubt sind, welche am Stützkörper 220 vorgesehen sind. Eine Relativbewegung des Stützkörpers 220 relativ zu benachbarten Wirbelkörpern 212 und 214 wird mittels zwischengeschalteter Bandscheibenkörper 224 und 225 realisiert.

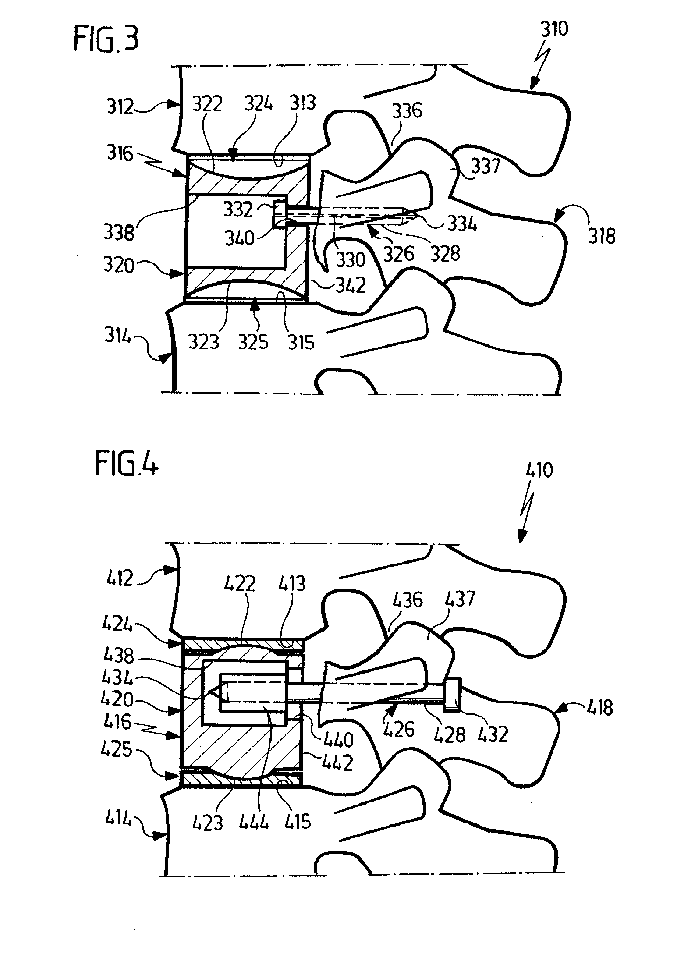

Ein drittes Ausführungsbeispiel eines Implantats ist in Figur 3 insgesamt mit dem Bezugszeichen 316 versehen. Es unterscheidet sich vom Implantat 216 im wesentlichen durch den anders geformten Stützkörper 320, der eine in ventraler Richtung offene Ausnehmung 338 aufweist.

Das Implantat 316 umfaßt zwei im wesentlichen zu den Bandscheibenkörpern 224 und 225 identische Bandscheibenkörper 324 und 325, die an Wirbelkörperflächen 313 beziehungsweise 315 benachbarter Wirbelkörper 312 beziehungsweise 314 anliegen. Eine eine dorsale Stützkörperwand 342 durchsetzende Bohrung 340 dient zur Aufnahme einer Knochenschraube 326, die von ventral nach dorsal in den Wirbelbogen 318 des verbliebenen Teils des Wirbels eingeschraubt ist.

Weist die Knochenschraube 326 einen in Längsrichtung verlaufenden Fluidkanal 330 auf, kann die Verankerung des Implantats zusätzlich verbessert werden, wenn über den Fluidkanal 330 beispielsweise Knochenzement eingespritzt wird. Darüber hinaus kann die Ausnehmung 338 mit Knochenzement gefüllt und entsprechend dem ursprünglichen Wirbelkörper modelliert werden.

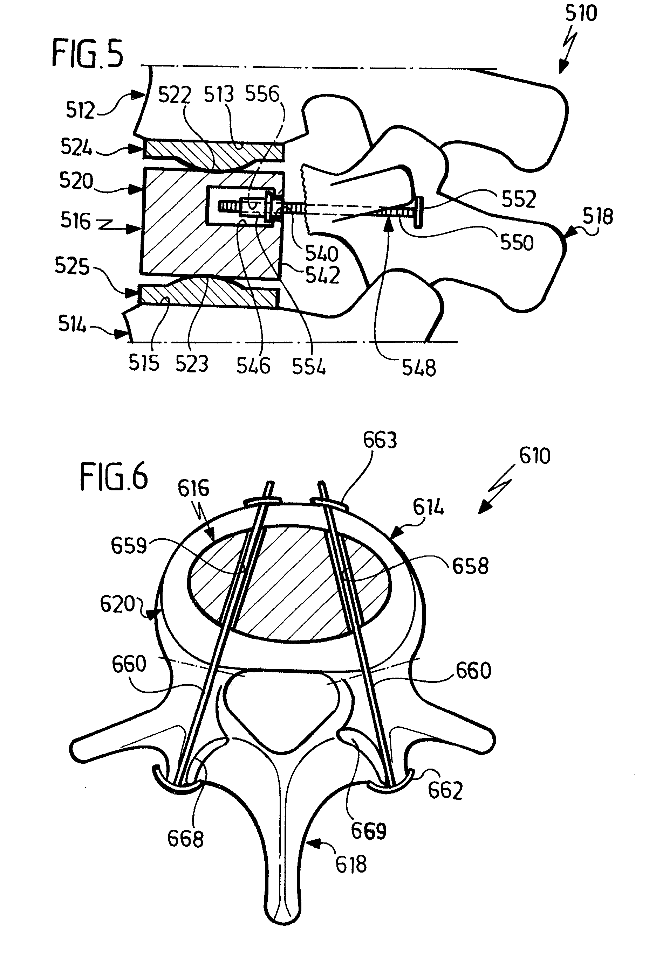

Ein viertes, insgesamt mit dem Bezugszeichen 416 versehenes Implantat ist in Figur 4 dargestellt. Ein im wesentlichen zylindrischer Stützkörper 420 weist an seinen Stirnflächen 422 beziehungsweise 423 schwach konvex/konkav gekrümmte Wölbungen auf, die mit korrespondierenden konkaven/konvexen Gleitflächen bildenden Ausnehmungen benachbarter Bandscheibenkörper 424 beziehungsweise 425 korrespondieren. Der mit einer in dorsaler Richtung geöffneten Ausnehmung 438 versehene Stützkörper 420 wird am zwischen zwei Wirbelkörpern 412 und 414 verbliebenen Rest eines Wirbels, im wesentlichen dem Wirbelbogen 418, mit einer Knochenschraube 426 fixiert, indem in die Ausnehmung 438 ein Dübel 444 eingesetzt wird, in den die Schraubenspitze 434 der den Wirbelbogen 418 von dorsal nach ventral durchsetzenden Knochenschraube 426 eindringt und den Dübel 444 aufspreizt.

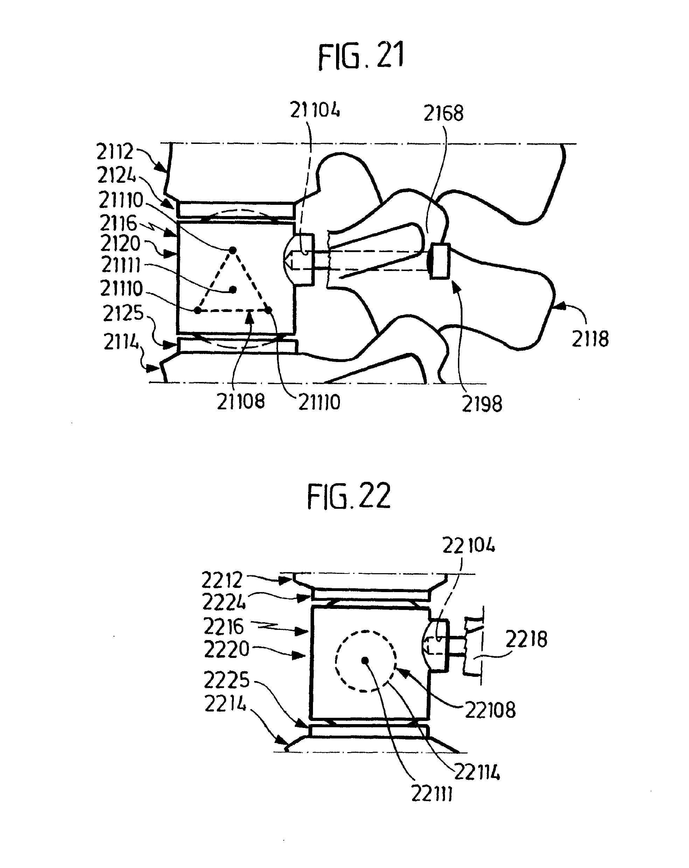

Der Dübel 444 und auch der Stützkörper 420 sind vorzugsweise aus röntgendurchlässigem oder röntgenhalbdurchlässigem Material hergestellt und mit einer röntgenundurchlässigen Zieleinrichtung, zum Beispiel einem Münsteraner Zielinstrument, versehen. Beispiele für mögliche Zielvorrichtungen werden im Zusammenhang mit den Figuren 21 und 22 näher erläutert.

Eine fünfte Variante eines erfindungsgemäßen Implantats ist in Figur 5 insgesamt mit den Bezugszeichen 516 versehen. Ein im wesentlichen zylindrischer Stützkörper 520 weist einen in seiner dorsalen Hälfte liegenden Hohlraum 546 auf, der von einer Bohrung in dorsaler Richtung eröffnet ist. Der Stützkörper 520 wird an verbliebenen Teilen eines Wirbelbogens 518 mit einem einen geriffelten Schaft 550 aufweisenden Stift 548 verbunden, der an einem Ende einen fest angebrachten Kopf 552 aufweist. Ein in den Hohlraum 546 eingesetzter Verriegelungsteller 554 ist mit einer Längsdurchbrechung 556 versehen, welche zu dem geriffelten Schaft 550 korrespondierende Rastelemente aufweist. Der Verriegelungsteller 554 wird somit auf dem Schaft 550 verrastet und der Stützkörper mit in bereits bekannter Weise angeordneten Bandscheibenkörpern 524 und 525 zwischen zwei Wirbelkörpern 512 beziehungsweise 514 festgelegt.

Alternativ kann statt des Stifts 548 auch eine Blindnietverbindung zwischen dem Stützkörper 520 und dem Wirbelbogen 518 mittels einem teilweise in den Hohlraum 546 eindringenden Blindniet hergestellt werden.

Alternativ ist auch eine Fixierung von ventral nach dorsal möglich, wenn sich der Hohlraum 546 in ventraler Richtung eröffnet.

In Figur 6 ist ein sechstes Ausführungsbeispiel eines insgesamt mit dem Bezugszeichen 616 versehenen Implantats abgebildet.

Es umfaßt einen im Querschnitt im wesentlichen elliptischen Stützkörper 620, der zwei Querbohrungen 658 beziehungsweise 659 aufweist, deren Längsachsen in dorsaler beziehungsweise ventraler Richtung einen spitzen Winkel einschließen oder parallel verlaufen. Zur Befestigung des Stützkörpers 620 an einem verbliebenen Wirbelbogen 618 wird ein Drahtstift oder ein Drahtseil 660 durch jeweils eine Querbohrung 658 beziehungsweise 659 hindurchgeschoben, wobei der Drahtstift 660 an beiden Enden tellerförmige Anschlagelemente 662 und 663 aufweist, von denen zumindest eines relativ zum Drahtstift 660 in einer Anlegestellung bewegbar ist. Der einseitig mit einem Anschlagelement 672 versehene Drahtstift 660 wird durch den verbliebenen Wirbelbogen 618 und die Querbohrung 658 hindurchgeschoben und ein zweites Anschlagelement 663 auf dem Drahtstift fixiert. Hierfür kann der Drahtstift 660 mit Rastelementen oder einem Gewinde versehen sein, wobei das Anschlagelement 663 korrespondierend zum Drahtstift 660 ausgebildet ist. Durch ein Verschieben des Anschlagelements 664 relativ zum Drahtstift 660 kann der Stützkörper 620 am verbliebenen Wirbelbogen 618 festgezurrt werden.

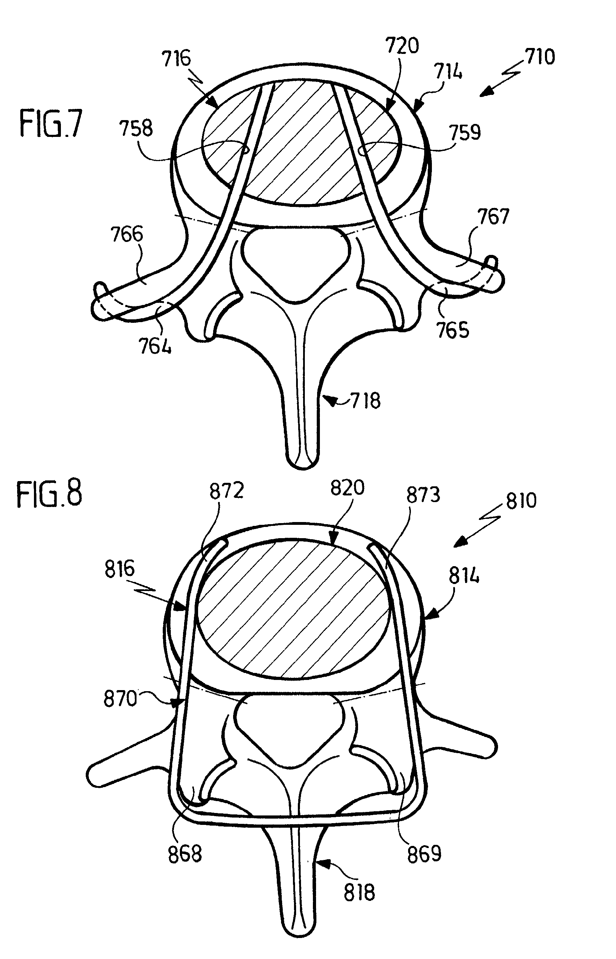

In den Figuren 7, 8 und 9 sind drei weitere Ausführungsbeispiele insgesamt mit den Bezugszeichen 716, 816 und 916 versehener Implantate dargestellt.

Das Implantat 716 umfaßt einen Stützkörper 720, der zwei in analoger Weise wie am Implantat 616 angeordnete Querbohrungen 758 und 759 aufweist. In diese ist jeweils ein Haken 764 beziehungsweise 765 eingesetzt, der in dorsaler Richtung aus dem Stützkörper 720 vorsteht und jeweils einen Dornfortsatz 766 beziehungsweise 767 eines verbliebenen Wirbelbogens 718 teilweise umschließt und auf diese Weise den Stützkörper 720 am Wirbelbogen 718 fixiert.

Das in Figur 8 dargestellte Implantat 816 umfaßt einen Stützkörper 820, der mit einer Klammer 870 an einer verbliebenen Struktur eines Wirbelbogens 816 festgelegt wird, indem die im wesentlichen U-förmige Klammer von dorsal über zwei Pedikel 868 beziehungsweise 869 mit freien Enden 872 beziehungsweise 873 den Stützkörper 820 umgreifend festgelegt wird. Klammerenden 872 beziehungsweise 873 sind aufeinander zu weisend gekrümmt und fixieren dadurch den Stützkörper 820 am verbliebenen Wirbelbogen 818.

Das Implantat 916 vereint Eigenschaften der Implantate 716 und 816. So weist ein Stützkörper 920 analog zum Stützkörper 720 Querbohrungen 958 und 959 auf, durch die freie Enden 872 und 873 einer im wesentlichen U-förmigen Klammer 970 von dorsal nach ventral durchgeschoben und aufeinander zu umgebogen werden. Wie die Klammer 870 umschließt die Klammer 970 Pedikel 868 und 869 eines verbliebenen Wirbelbogens 918 eines teilweise entfernten Wirbels. Die beiden Haken 764 und 765 sowie die beiden Klammern 870 beziehungsweise 970 können beispielsweise aus Nitinol hergestellt sein.

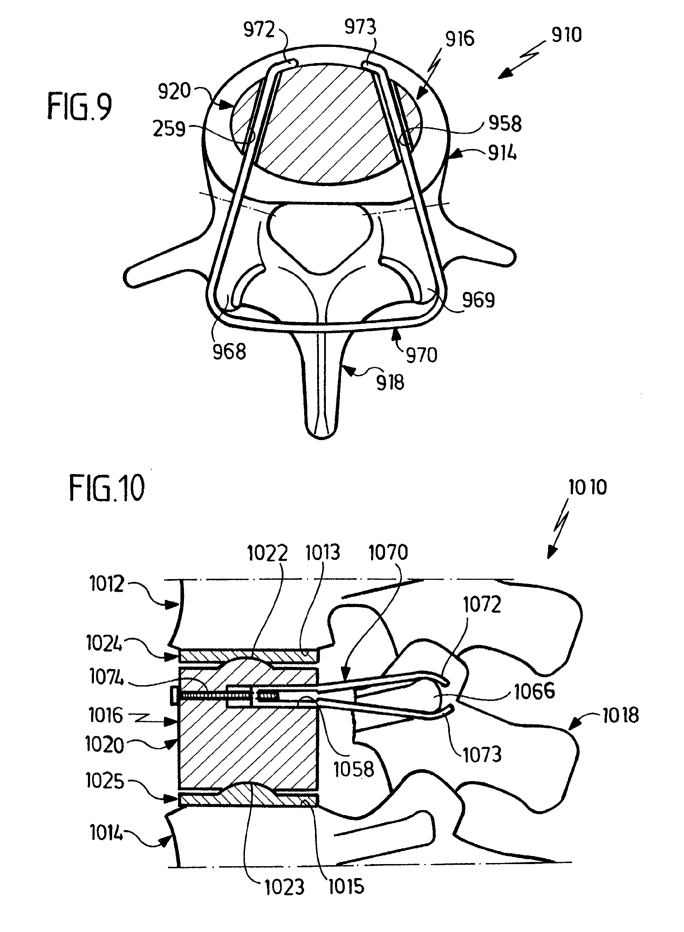

Figur 10 zeigt ein zehntes Ausführungsbeispiel eines insgesamt mit dem Bezugszeichen 1016 versehenen Implantats.

Es weist wie alle oben beschriebenen Implantate einen Stützkörper 1020 und zwei an diesen benachbart angrenzende Bandscheibenkörper 1024 und 1025 auf, welche wiederum an von einem teilweise zerstörten Wirbelkörper getrennte Wirbelkörper 1012 beziehungsweise 1014 angrenzen.

Der Stützkörper 1020 weist eine oder zwei Querbohrungen 1058 auf, deren Durchmesser sich in dorsaler Richtung einstufig erweitert. In die Querbohrung ist von dorsal eine längliche Klammer 1070 eingesetzt, deren freie Klammerenden 1072 und 1073 aufeinander zu weisend einen Dornfortsatz 1066 eines Wirbelbogens 1018 teilweise umgreifen. Durch den einen kleineren Durchmesser aufweisenden Abschnitt der Querbohrung 1058 ragt eine mit ihrem Kopf am ventralen Ende der Querbohrung 1058 anschlagende Schraube 1074, die mit der Klammer 1070 im Bereich deren ventralen Endes verschraubt ist.

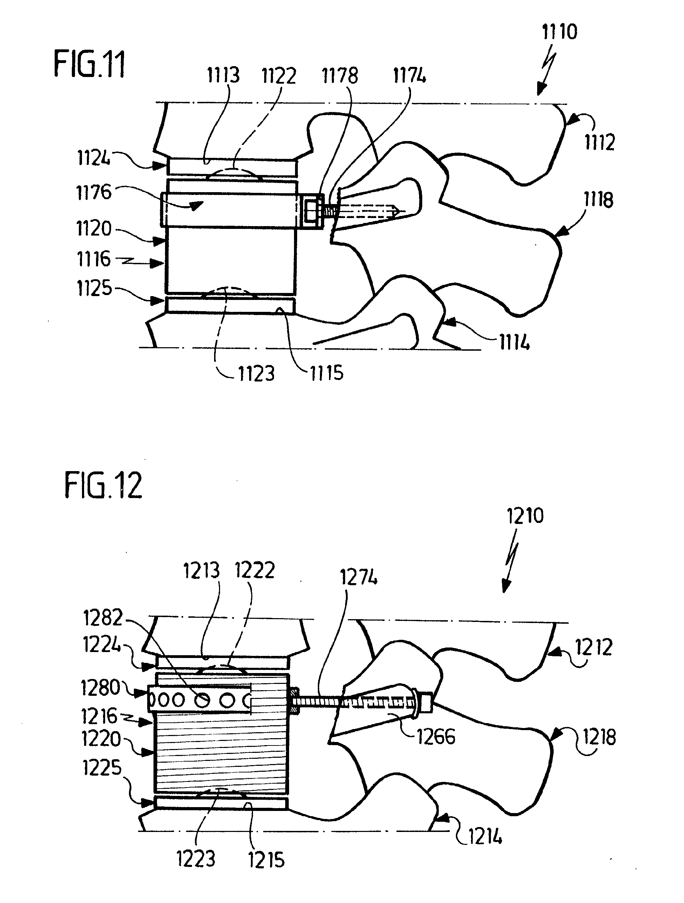

Ein elftes Ausführungsbeispiel eines Implantats 1116 ist in Figur 11 abgebildet.

Ein zwischen zwei Bandscheibenkörper 1124 und 1125 eingesetzter Stützkörper 1120 wird von einer Schelle 1176 umgeben, die einseitig eine Schraubenkopfaufnahme 1178 aufweist, so daß eine Schraube 1174 von einer dorsalen Seite des Stützkörpers 1120 in dorsaler Richtung in einen verbliebenen Teil eines zwischen zwei Wirbelkörpern 1112 und 1114 befindlichen Wirbelbogens 1118 eingeschraubt werden kann. Die Schelle 1176 ist in einer Anlegestellung am Stützkörper 1120 längsverschieblich gelagert, in einer Fixierstellung relativ zum Stützkörper 1120 festgelegt.

In Figur 12 ist ein insgesamt mit dem Bezugszeichen 1216 versehenes Implantat dargestellt, das einen mit einem Außengewinde versehenen zylindrischen Stützkörper 1220 aufweist, dessen Stirnflächen an benachbarten Bandscheibenkörpern 1224 und 1225 anliegen, die eine Relativbewegung des Stützkörpers 1220 relativ zu an den beiden Bandscheibenkörpern 1224 und 1225 angrenzenden Wirbelkörpern 1212 und 1214 ermöglichen. Der Stützkörper 1220 wird an einem verbliebenen Rest eines Wirbels fixiert, indem eine Schraube 1274 von dorsal nach ventral mit einem Wirbelbogen 1218 verschraubt wird beziehungsweise diesen durchsetzt und in eine von mehreren mit einem korrespondierenden Gewinde versehenen Radialbohrungen eines auf den Stützkörper 1220 aufgeschraubten Lagerring eingeschraubt wird. Eine optimale Anpassung der Position des Stützkörpers 1220 relativ zum Wirbelbogen 1218 wird ermöglicht, indem zunächst der Ring 1280 durch Verdrehen um den Stützkörper 1220 in die gewünschte Position gebracht wird, und dann die Schraube 1274 in eine der mit einem Gewinde versehenen Radialbohrungen 1282 eingeschraubt wird.

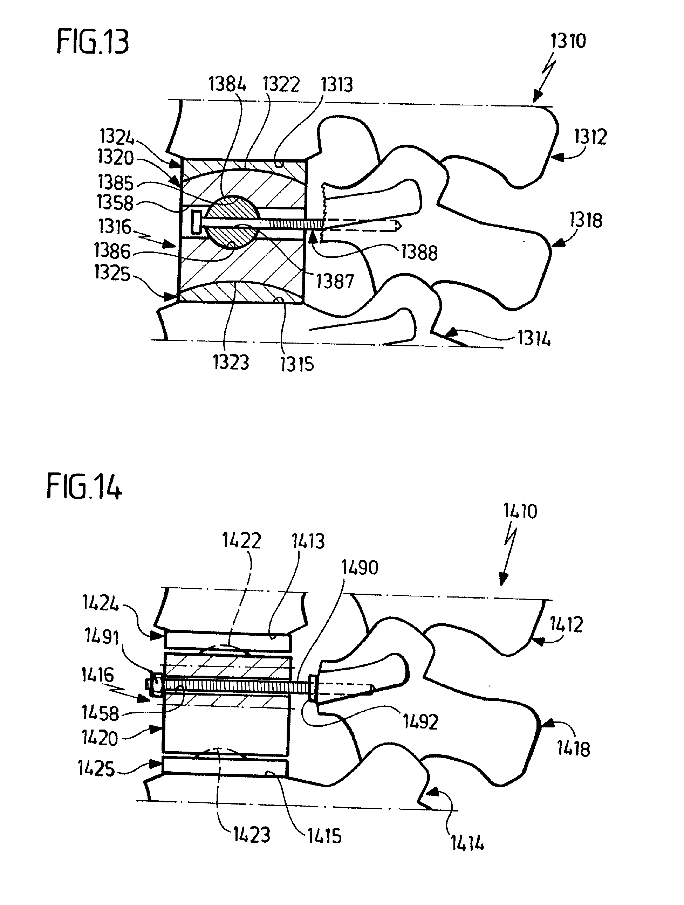

Mit einem insgesamt mit dem Bezugszeichen 1316 versehenen und in Figur 13 dargestellten dreizehnten Ausführungsbeispiel eines Implantats ist ebenfalls eine optimale Anpassung eines Stützkörpers 1320 an einen verbliebenen Teil eines Wirbels möglich. Zu diesem Zweck ist in eine Querbohrung 1358 eine Kugel 1384 eingesetzt und vollständig frei rotierbar gelagert in korrespondierenden Kugelflächen 1385 beziehungsweise 1386 der Querbohrung 1358. Die Kugel 1384 wiederum weist eine durch ihren Mittelpunkt verlaufende Zentralbohrung 1387 auf, in die ein gewindefreier Schaftabschnitt einer Knochenschraube 1388 eingeführt ist. Durch diese besondere Lagerung der Knochenschraube 1388 mittels der Kugel 1384 läßt sich eine Relativposition zwischen dem Stützkörper 1320 und einem Wirbelbogen 1318 nahezu beliebig einstellen. Eine Relativbewegung zwischen dem Stützkörper 1320 und benachbarten Wirbelkörpern 1312 und 1314 wird über jeweils dazwischen liegende Bandscheibenkörper 1324 und 1325 ermöglicht.

Durch eine geeignete Materialwahl der Kugel 1384 ist eine genaue Positionsbestimmung der Knochenschraube 1388 beim Einsetzen des Implantats 1316 unter Röntgenkontrolle möglich.

Ein insgesamt mit dem Bezugszeichen 1416 versehenes Implantat ist in Figur 14 dargestellt. Es wird an einem verbliebenen Teil eines Wirbels festgelegt, indem ein Stützkörper 1420 mit einer Querbohrung 1458 versehen wird, durch welche ein Gewindestab 1490 durchgesteckt wird. Letzterer ist in zwei Gewindeabschnitte unterteilt, die durch einen ringförmigen, auf einen der Abschnitte aufgeschraubten Anschlag 1492 getrennt sind. Durch den Anschlag 1492 wird verhindert, daß der Gewindestab 1490 beliebig tief in einen Wirbelbogen 1418 eindringen kann. Mittels einer Mutter 1491, die mit dem die Querbohrung 1458 durchdringenden Gewindeabschnitt des Gewindestabs 1490 verschraubbar ist, wird der Stützkörper 1420 in seiner Position fixiert.

Eine Relativbewegung zwischen dem Stützkörper 1420 und benachbarten Wirbelkörpern 1412 und 1414 wird mittels zwischengeschobener Bandscheibenkörper 1424 und 1425 ermöglicht.

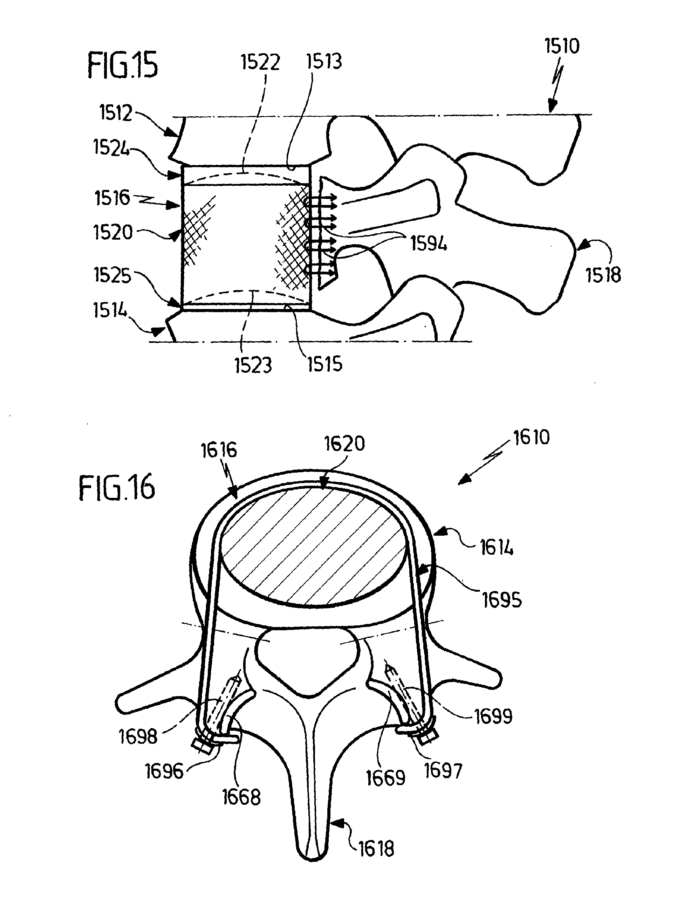

Bei einem in Figur 15 dargestellten fünfzehnten Ausführungsbeispiel eines insgesamt mit dem Bezugszeichen 1516 versehenen Implantats wird eine Verbindung zwischen einem Stützkörper 1520 und einem verbliebenen Wirbelbogen 1518 eines teilweise entfernten Wirbels mittels eines beliebigen Nahtmaterials 1594 realisiert. Der Stützkörper 1520 weist vorzugsweise Durchbrechungen zum Durchführen des Nahtmaterials 1594 auf, welches beispielsweise ein Faden oder ein Draht sein kann. Insbesondere bei einem hohlen Stützkörper 1520 mit einer Hülle aus einem Drahtgeflecht, läßt sich so auf einfache Weise der Stützkörper 1520 am Wirbelbogen 1518 fixieren.

In ähnlicher Weise wie im Zusammenhang mit dem in Figur 8 beschriebenen Implantat 816 wird ein Stützkörper 1620 eines in Figur 16 dargestellten Implantats 1616 an verbleibenden Teilen eines teilweise zerstörten Wirbels fixiert, indem ein Band 1695 oder ein Flachband um den Stützkörper 1620 herumgelegt wird und freie Enden 1696 und 1697 des Bandes 1695 mit jeweils einer Pedikelschraube 1698 und 1699 in jeweils einen Pedikel 1668 und 1669 befestigt werden.

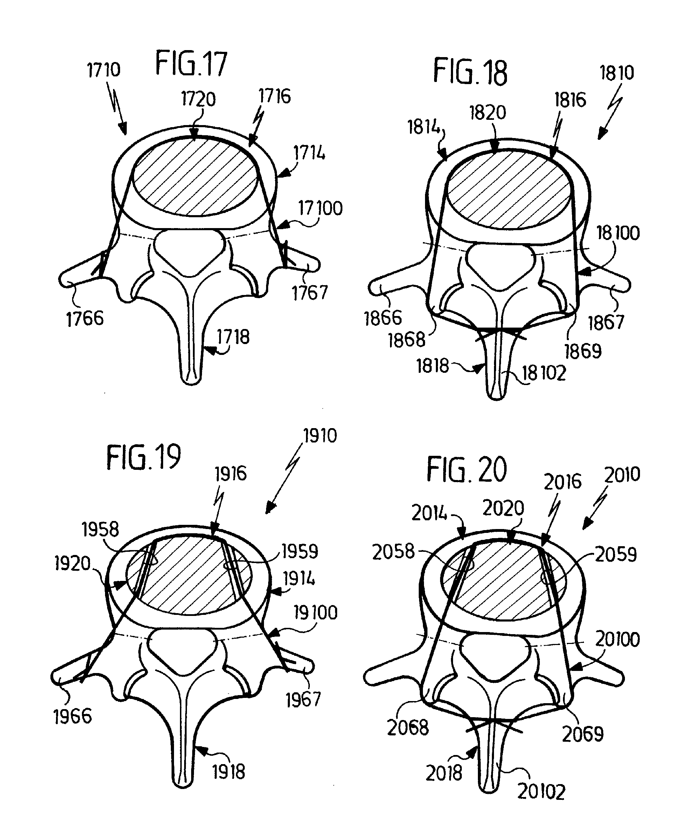

In den Figuren 17 bis 20 sind jeweils Implantate dargestellt, die mittels eines Fadens oder Drahts an Resten eines verbliebenen Wirbels fixiert werden, beispielsweise durch Nähen oder Verknoten.

Ein siebzehntes, insgesamt mit dem Bezugszeichen 1716 versehenes Implantat wird mit einem Faden 17100 fixiert, indem dieser um einen Stützkörper 1720 herumgelegt und mit seinen freien Enden jeweils an einem Querfortsatz 1766 beziehungsweise 1767 eines Wirbelbogens 1718 durch Nähen oder Knoten festgelegt wird.

Analog zum Implantat 1716 ist das in Figur 18 dargestellte und insgesamt mit dem Bezugszeichen 1816 versehene Implantat ebenfalls mit einem Faden 18100 an einem Wirbelbogen 1818 fixiert, allerdings nicht an seitlich abstehenden Querfortsätzen 1866 beziehungsweise 1867, sondern an einem in dorsaler Richtung abstehenden Dornfortsatz 18102. Der Faden 18100 ist um einen Stützkörper 1820 herumgelegt und am Dornfortsatz 18102 vernäht beziehungsweise verknotet.

Das in Figur 19 insgesamt mit dem Bezugszeichen 1916 versehene neunzehnte Ausführungsbeispiel eines erfindungsgemäßen Implantats wird mit einem Faden 19100 analog dem Implantat 1716 an einem verbliebenen Wirbelbogen 1918 fixiert, indem freie Enden des Fadens 19100 um seitlich abstehende Querfortsätze 1966 beziehungsweise 1967 herumgeschlungen und mit diesen verknotet beziehungsweise vernäht werden. Ein Unterschied zum Implantat 1716 besteht allerdings darin, daß ein Stützkörper 1920 mit zwei relativ zueinander geneigten Querbohrungen 1958 und 1959 versehen ist, durch die der Faden 19100 hindurchgeführt ist, so daß er den Stützkörper 1920 nicht wie beim siebzehnten Ausführungsbeispiel vollständig umgibt, sondern teilweise durchsetzt.

Das in Figur 20 dargestellte zwanzigste Ausführungsbeispiel eines insgesamt mit dem Bezugszeichen 2016 versehenen Implantats stellt eine Kombination des achtzehnten und neunzehnten Ausführungsbeispiels dar. Ein Faden 20100 durchsetzt zwei Querbohrungen 2058 und 2059 eines Stützkörpers 2020, ist um zwei abstehende Pedikel 2068 und 2069 herumgeführt und schließlich mit einem in dorsaler Richtung abstehenden Dornfortsatz 20102 verknotet oder vernäht.

In den Figuren 21 und 22 sind zwei mögliche Ausgestaltungen von Zielvorrichtungen dargestellt.

Figur 21 zeigt ein einundzwanzigstes Ausführungsbeispiel eines insgesamt mit dem Bezugszeichen 2116 versehenen Implantats, das im wesentlichen dem in Figur 2 dargestellten entspricht. Zur Vereinfachung sind lediglich ein mit einer Gewindebohrung 21104 versehener Stützkörper 2120 sowie die daran anliegenden Bandscheibenkörper 2124 und 2125 eingezeichnet.

Eine insgesamt mit dem Bezugszeichen 21108 versehene Zielvorrichtung umfaßt drei radial vom Stützkörper 2120 abstehende, als Eckpunkte eines gleichseitigen Dreiecks angeordnete, aus einem röntgenundurchlässigen Material hergestellte Metallstifte 21110. Im Schwerpunkt des gleichschenkligen Dreiecks ist ein weiterer Metallstift 21111 angeordnet. Die insgesamt vier Metallstifte 21110 und 21111 können beliebig am Stützkörper 2120 angeordnet sein. Sie dienen dazu, ein mit dem Stützkörper 2120 zu verbindendes Fixierelement gezielt relativ zu ersterem zu positionieren und einzuführen.

Figur 22 zeigt eine weitere Variante einer Zielvorrichtung 22108, die im wesentlichen aus einem im Zentrum eines Metallrings 22114 angeordneten Metallstift 22111 besteht. Die Zielvorrichtung 22108 kann an einem beliebigen der oben beschriebenen Implantate angeordnet werden. In Figur 22 ist sie beispielhaft an einem bereits im Zusammenhang mit der Figur 2 beschriebenen Implantat angeordnet, das insgesamt mit dem Bezugszeichen 2216 versehen ist, einen Stützkörper 2220 und zwei daran anliegende Bandscheibenkörper 2224 und 2225 umfaßt.

Sowohl die im Zusammenhang mit der Figur 21 als auch die im Zusammenhang mit der Figur 22 vorgestellten Zieleinrichtungen 21108 und 22108 ermöglichen es, aufgrund einer Bestrahlung mittels Röntgenstrahlen eine eindeutige und genaue Positionierung des Stützkörpers relativ zu einem nicht dargestellten, aber jeweils im Zusammenhang mit den Ausführungsbeispielen eins bis zwanzig beschriebenen Fixierelement zu gewährleisten.

## Patentansprüche

1. Implantat (116, 216, 316, 416, 516, 616, 716, 816, 916, 1016, 1116, 1216, 1316, 1416, 1516, 1616, 1716, 1816, 1916, 2016, 2116, 2216) zum Ersetzen mindestens eines mindestens teilweise zerstörten Wirbelkörpers eines Wirbels und zum Einsetzen zwischen einem ersten und einem zweiten Wirbelkörper (112, 114) einer Wirbelsäule (110, 210, 310, 410, 510, 610, 710, 810, 910, 1010, 1110, 1210, 1310, 1410, 1510, 1610, 1710, 1810, 1910, 2010, 2110, 2210), mit mindestens einem Bewegungselement (124, 125; 224, 225; 324, 325; 424, 425; 524, 525; 1024, 1025; 1124, 1125; 1224, 1225; 1324, 1325; 1424, 1425; 1524, 1525; 2124, 2125; 2224, 2225) und mindestens einem Stützelement (120; 220; 320; 420; 520; 620; 720; 820; 920; 1020; 1120; 1220; 1320; 1420; 1520; 1620; 1720; 1820; 1920; 2020; 2120; 2220), wobei das mindestens eine Bewegungselement (124, 125; 224, 225; 324, 325; 424, 425; 524, 525; 1024, 1025; 1124, 1125; 1224, 1225; 1324, 1325; 1424, 1425; 1524, 1525; 2124, 2125; 2224, 2225) dem Stützelement (120; 220; 320; 420; 520; 620; 720; 820; 920; 1020; 1120; 1220; 1320; 1420; 1520; 1620; 1720; 1820; 1920; 2020; 2120; 2220) und dem ersten und/oder dem zweiten Wirbelkörper (112, 114) zugeordnet ist zum Ermöglichen einer Bewegung des mindestens einen Stützelements (120; 220; 320; 420; 520; 620; 720; 820; 920; 1020; 1120; 1220; 1320; 1420; 1520; 1620; 1720; 1820; 1920; 2020; 2120; 2220) relativ zu dem ersten und/oder dem zweiten Wirbelkörper (112, 114), **dadurch gekennzeichnet, daß** mindestens eine mit mindestens einem Wirbelbogen (118; 218; 318; 418; 518; 618; 718; 818; 918; 1018; 1118; 1218; 1318; 1418; 1518; 1618; 1718; 1818; 1918; 2018) der Wirbelsäule verbundene Fixiervorrichtung (126; 298; 326, 338; 426, 444, 438; 546, 548, 554; 660, 662, 663; 764, 765; 870; 970; 1073, 1074; 1174, 1176, 1178; 1274, 1280; 1386, 1388; 1490, 1491, 1492; 1594; 1695, 1698, 1699; 17100; 18100; 19100; 20100) vorgesehen ist zum Fixieren des mindestens einen Stützelements (120; 220; 320; 420; 520; 620; 720; 820; 920; 1020; 1120; 1220; 1320; 1420; 1520; 1620; 1720; 1820; 1920; 2020; 2120; 2220) an dem mindestens einen Wirbelbogen (118; 218; 318; 418; 518; 618; 718; 818; 918; 1018; 1118; 1218; 1318; 1418; 1518; 1618; 1718; 1818; 1918; 2018).

2. Implantat nach Anspruch 1, **dadurch gekennzeichnet, daß** die mindestens eine Fixiervorrichtung (126; 298; 326, 338; 426, 444, 438; 546, 548, 554; 660, 662, 663; 764, 765; 870; 970; 1073, 1074; 1174, 1176, 1178; 1274, 1280; 1386, 1388; 1490, 1491, 1492; 1594; 1695, 1698, 1699; 17100; 18100; 19100; 20100) mit dem mindestens einen Stützelement (120; 220; 320; 420; 520; 620; 720; 820; 920; 1020; 1120; 1220; 1320; 1420; 1520; 1620; 1720; 1820; 1920; 2020; 2120; 2220) und dem mindestens einen Wirbelbogen (118; 218; 318; 418; 518; 618; 718; 818; 918; 1018; 1118; 1218; 1318; 1418; 1518; 1618; 1718; 1818; 1918; 2018) des teilweise zerstörten Wirbels verbindbar ist.

3. Implantat nach einem der voranstehenden Ansprüche, **dadurch gekennzeichnet, daß** das mindestens eine Stützelement (220; 320; 420; 520; 620; 720; 820; 920; 1020; 1120; 1220; 1320; 1420; 1620; 1720; 1820; 1920; 2020) im wesentlichen biegesteif ist.

4. Implantat nach einem der voranstehenden Ansprüche, **dadurch gekennzeichnet, daß** das mindestens eine Stützelement (120; 320; 420; 520; 1520; 2220; 2320) einen Hohlraum (338; 438; 546) aufweist.

5. Implantat nach einem der voranstehenden Ansprüche, **dadurch gekennzeichnet, daß** das mindestens eine Stützelement (120; 320; 420; 520; 1520; 2220; 2320) eine mit einer aushärtbaren Substanz füllbare Hülle umfaßt.

6. Implantat nach Anspruch 5, **dadurch gekennzeichnet, daß** die Hülle (120; 1520; 2220; 2320) biegeschlaff und/oder flexibel ist.

7. Implantat nach einem der Ansprüche 5 oder 6, **dadurch gekennzeichnet, daß** die aushärtbare Substanz Knochenzement ist.

8. Implantat nach einem der voranstehenden Ansprüche, **dadurch gekennzeichnet, daß** die mindestens eine Fixiervorrichtung mindestens ein Fixierelement (126; 298; 326, 338; 426, 444, 438; 546, 548, 554; 660, 662, 663; 764, 765; 870; 970; 1073, 1074; 1174, 1176, 1178; 1274, 1280; 1386, 1388; 1490, 1491, 1492; 1594; 1695, 1698, 1699; 17100; 18100; 19100; 20100) umfaßt.

9. Implantat nach Anspruch 8, **dadurch gekennzeichnet, daß** das mindestens eine Fixierelement mindestens eine Schraube (126; 326; 426; 1074; 1174; 1274; 1388; 1698, 1699), eine Pedikelschraube (298), einen Stift (548; 660; 1490), einen Haken (764, 765; 870; 970; 1073) oder einen Dübel (444) umfaßt.

10. Implantat nach Anspruch 9, **dadurch gekennzeichnet, daß** der mindestens eine Dübel (444) in einen Hohlraum (438) des mindestens einen Stützelements (420) einsetzbar und die mindestens eine Schraube (426) mit dem mindestens einen Dübel (444) verbindbar ist.

11. Implantat nach einem der Ansprüche 8 bis 10, **dadurch gekennzeichnet, daß** das mindestens eine Fixierelement einen Blindniet (554) umfaßt.

12. Implantat nach einem der Ansprüche 8 bis 11, **dadurch gekennzeichnet, daß** das mindestens eine Fixierelement (444; 554) spreizbar ist und daß es insbesondere einen spreizbaren Stift umfaßt.

13. Implantat nach einem der voranstehenden Ansprüche, **dadurch gekennzeichnet, daß** die mindestens eine Fixiervorrichtung mindestens einen Drahtstift (550; 660) mit mindestens zwei relativ zum mindestens einen Drahtstift (550; 660) in einer Fixierstellung in Längsrichtung des mindestens einen Drahtstifts (550; 660) festlegbaren Anschlagelementen (552, 554; 662, 663) umfaßt.

14. Implantat nach Anspruch 13, **dadurch gekennzeichnet, daß** mindestens eines der mindestens zwei Anschlagelemente (662, 663) in einer Anlegestellung relativ zum mindestens einen Drahtstift (550; 660) bewegbar ist.

15. Implantat nach einem der Ansprüchen 13 oder 14, **dadurch gekennzeichnet, daß** der mindestens eine Drahtstift (550; 660) mit einem Gewinde versehen ist.

16. Implantat nach einem der Ansprüche 13 bis 15, **dadurch gekennzeichnet, daß** der mindestens eine Drahtstift (660) mit Rastelementen versehen ist.

17. Implantat nach einem der Ansprüche 13 bis 16, **dadurch gekennzeichnet, daß** die mindestens zwei Anschlagelemente (552, 554; 662, 663) tellerförmig sind.

18. Implantat nach einem der Ansprüche 8 bis 17, **dadurch gekennzeichnet, daß** das mindestens eine Fixierelement (764, 765; 870; 970; 1070; 1695) in Richtung auf den Wirbelbogen (118; 218; 318; 418; 518; 618; 718; 818; 918; 1018; 1118; 1218; 1318; 1418; 1518; 1618; 1718; 1818; 1918; 2018) hakenförmig gekrümmt ist und mindestens einen am Wirbelbogen (118; 218; 318; 418; 518; 618; 718; 818; 918; 1018; 1118; 1218; 1318; 1418; 1518; 1618; 1718; 1818; 1918; 2018) angeordneten Pedikel (868, 869; 968, 969; 1068; 1668, 1669) und/oder Dornfortsatz und/oder Querfortsatz (766, 767) im eingesetzten Zustand mindestens teilweise umschließt.

19. Implantat nach Anspruch 18, **dadurch gekennzeichnet, daß** das mindestens eine hakenförmig gekrümmte Fixierelement den mindestens einen Pedikel und/oder Dornfortsatz und/oder Querfortsatz vollständig umgibt.

20. Implantat nach einem der voranstehenden Ansprüche, **dadurch gekennzeichnet, daß** die mindestens eine Fixiervorrichtung ein mit einer Befestigungselementaufnahme versehenes Band (1695) umfaßt.

21. Implantat nach einem der voranstehenden Ansprüche, **dadurch gekennzeichnet, daß** die mindestens eine Fixiervorrichtung eine das mindestens eine Stützelement (1120; 1220) umschließende Schelle (1176; 1280) umfaßt.

22. Implantat nach Anspruch 21, **dadurch gekennzeichnet, daß** die Schelle (1176; 1280) in einer Anlegestellung relativ zum mindestens einen Stützelement (1120; 1220) verschiebbar und in einer Fixierstellung relativ zum mindestens einen Stützelement (1120; 1220) festlegbar ist.

23. Implantat nach einem der voranstehenden Ansprüche, **dadurch gekennzeichnet, daß** die mindestens eine Fixiervorrichtung mindestens ein bewegliches Lagerelement (1384) zum beliebigen Verschwenken des Fixierelements (1388) relativ zum mindestens einen Stützelement (1320) umfaßt.

24. Implantat nach Anspruch 23, **dadurch gekennzeichnet, daß** das mindestens eine Lagerelement (1384) kugelförmig ist.

25. Implantat nach einem der Ansprüche 23 oder 24, **dadurch gekennzeichnet, daß** das mindestens eine Lagerelement (1384) einen Absorptionskoeffizient für Röntgenstrahlen aufweist, der von dem des mindestens einen Stützelements (1320) abweicht.

26. Implantat nach einem der Ansprüche 23 bis 25, **dadurch gekennzeichnet, daß** das mindestens eine Lagerelement eine mit einer Zentralbohrung versehene Kugel (1384) umfaßt.

27. Implantat nach einem der Ansprüche 8 bis 26, **dadurch gekennzeichnet, daß** das mindestens eine Fixierelement ein Nahtmaterial (1594; 17100; 18100; 19100; 20100) umfaßt, das mit dem mindestens einen Wirbelbogen (1518; 1718; 1818; 1918; 2018) vernäh- oder verknotbar ist.

28. Implantat nach Anspruch 27, **dadurch gekennzeichnet, daß** die mindestens eine Fixiervorrichtung mindestens einen Knochenanker zum Fixieren des Nahtmaterials (1594; 17100; 18100; 19100; 20100) am Wirbelbogen (1518; 1718; 1818; 1918; 2018) umfaßt.

29. Implantat nach einem der Ansprüche 8 bis 28, **dadurch gekennzeichnet, daß** an dem mindestens einen Fixierelement (1490) ein auf einem Gewinde bewegbarer Anschlag (1492) vorgesehen ist.

30. Implantat nach einem der Ansprüche 8 bis 29, **dadurch gekennzeichnet, daß** das mindestens eine Fixierelement (126; 326) einen Fluidkanal (130; 330) zum Einführen eines Fluids in einen Hohlraum des mindestens einen Stützelements (120) und/oder zum Einführen eines aushärtbaren Fluids zum Verankern des Fixierelements (328) im Wirbelbogen umfaßt.

31. Implantat nach einem der Ansprüche 8 bis 30, **dadurch gekennzeichnet, daß** die mindestens eine Fixiervorrichtung mindestens eine am mindestens einen Stützelement vorgesehene Fixierelementaufnahme (2104; 340; 438; 546; 658, 659; 758, 759; 958, 959; 1058; 1178; 1282; 1358; 1458; 1958, 1959; 2058, 2059; 21104; 22104) zum Aufnehmen des mindestens einen, am mindestens einen Wirbelbogen (218; 318, 418, 518, 618, 718; 918, 1018, 1118, 1218, 1318, 1418; 1918; 2018; 2118; 2218) befestigbaren Fixierelements (298; 326, 426; 548; 660; 764, 765; 970; 1070, 1074; 1174; 1274; 1388; 1490; 19100, 20100; 2198; 2298) umfaßt.

32. Implantat nach Anspruch 31, **dadurch gekennzeichnet, daß** die mindestens eine Fixierelementaufnahme eine Gewindebohrung (2104; 1282; 21104; 22104) umfaßt.

33. Implantat nach einem der voranstehenden Ansprüche, **dadurch gekennzeichnet, daß** das mindestens eine Stützelement (320) eine vom mindestens einen Wirbelbogen (318) weg weisende Ausnehmung (338) umfaßt.

34. Implantat nach Anspruch 33, **dadurch gekennzeichnet, daß** die Ausnehmung (338) einen Anschlag für das mindestens eine Fixierelement (326) umfaßt.

35. Implantat nach einem der voranstehenden Ansprüche, **dadurch gekennzeichnet, daß** das mindestens eine Bewegungselement (124, 125) eine mit einer dauerhaft fließfähigen Substanz gefüllte Hülle umfaßt.

36. Implantat nach einem der voranstehenden Ansprüche, **dadurch gekennzeichnet, daß** das mindestens eine Bewegungselement (224, 225; 324, 325; 424, 425; 524, 525; 624, 625; 724, 725; 824, 825; 924, 925; 1024, 1025; 1124, 1125; 1224, 1225; 1324, 1325; 1424, 1425; 1524, 1525; 1624, 1625; 1724, 1725; 1824, 1825; 1924, 1925; 2024, 2025) aus einer teilweise elastischen Substanz hergestellt ist.

37. Implantat nach einem der voranstehenden Ansprüche, **dadurch gekennzeichnet, daß** das mindestens eine Bewegungselement (224, 225; 324, 325; 424, 425; 524, 525; 1024, 1025; 1124, 1125; 1224, 1225; 1324, 1325; 1424, 1425; 1524, 1525; 2124, 2125; 2224, 2225) konkav oder konvex gekrümmte kalottenförmige Bereiche zur Bildung von Gleitflächen für daran anliegende Bereiche des Stützkörpers (220; 320; 420; 520; 1020; 1120; 1220; 1320; 1420; 1520; 2120; 2220) umfaßt.

38. Implantat nach einem der voranstehenden Ansprüche, **dadurch gekennzeichnet, daß** eine dem Implantat (116; 216; 316; 416; 516; 616; 716; 816; 916; 1016; 1116; 1216; 1316; 1416; 1516; 1616; 1716; 1816; 1916; 2016; 2116; 2216) zugeordnete Zielvorrichtung (21108; 22108) vorgesehen ist.

39. Implantat nach Anspruch 38, **dadurch gekennzeichnet, daß** die Zielvorrichtung (21108; 22108) am Implantat (116; 216; 316; 416; 516; 616; 716; 816; 916; 1016; 1116; 1216; 1316; 1416; 1516; 1616; 1716; 1816; 1916; 2016; 2116; 2216) angeordnet ist.

40. Implantat nach einem der Ansprüche 38 oder 39, **dadurch gekennzeichnet, daß** die Zielvorrichtung (21108; 22108) aus einem Material hergestellt ist, das einen Absorptionskoeffizient für Röntgenstrahlen aufweist, der von dem des mindestens einen Stützelements (120; 220; 320; 420; 520; 620; 720; 820; 920; 1020; 1120; 1220; 1320; 1420; 1520; 1620; 1720; 1820; 1920; 2020).

41. Implantat nach einem der Ansprüche 38 bis 40, **dadurch gekennzeichnet, daß** das Implantat (116; 216; 316; 416; 516; 616; 716; 816; 916; 1016; 1116; 1216; 1316; 1416; 1516; 1616; 1716; 1816; 1916; 2016; 2116; 2216) im wesentlichen aus für Röntgenstrahlen durchlässigem oder teilweise durchlässigem Material hergestellt ist.
